# FASCICULE DE BREVET EUROPEEN

(11) **EP 1 524 945 B1**
(45) Date de publication et mention de la délivrance du brevet: **26.08.2009**
(21) Numéro de dépôt: 03750795.1
(22) Date de dépôt: 10.07.2003
(51) Int. Cl.: A61B 17/70

(54) **DISPOSITIF D'ANCRAGE OSSEUX AVEC ARTICULATION SPHERIQUE**
KNOCHENVERANKERUNGSVORRICHTUNG MIT KUGELGELENK
BONE ANCHORING DEVICE WITH SPHERICAL ARTICULATION

(30) Priorité: 12.07.2002 FR 0208838
(43) Date de publication de la demande: 27.04.2005
(73) Titulaire: SCIENT'X, 78284 Guyancourt (FR)
(72) Inventeur: CARLI, Olivier, 78284 Guyancourt (FR)
(74) Mandataire: Thibault, Jean-Marc
(86) Numéro de dépôt international: PCT/FR2003/002167
(87) Numéro de publication internationale: WO 2004/006791

(56) Documents cités:
- WO-A-95/10239
- FR-A- 2 799 947
- US-A- 5 628 740
- US-A- 5 725 528

## Description

La présente invention concerne le domaine technique de l'ostéosynthèse notamment du rachis et elle vise plus précisément un dispositif d'ancrage dans les vertèbres utilisé dans les systèmes d'ostéosynthèse pour le rachis.

L'objet de l'invention vise plus précisément un dispositif d'ancrage osseux, tel qu'une vis d'ancrage conçue pour présenter une angulation relative avec un système d'ostéosynthèse mettant en oeuvre une tige de liaison par exemple.

Une des applications connue de l'objet de l'invention concerne les systèmes conçus pour corriger et stabiliser le rachis et pour faciliter la fusion osseuse à différents niveaux du rachis. Selon une telle application, un tel système comporte un élément de liaison intervertébrale tel qu'une tige ou une plaque apte à être cintrée et disposée le long du rachis tout en étant maintenue en position par des vis implantées dans les vertèbres pour suivre la courbure de la région du rachis appareillé. Ainsi, pour respecter l'anatomie du rachis, la tige de liaison doit être conformée pour présenter des angulations importantes notamment pour son montage en relation des vertèbres lombaires et sacrées.

Pour autoriser de telles conformations de la tige tout en assurant son blocage efficace par rapport aux vis d'ancrage, il est connu d'équiper les vis d'ancrage d'une articulation sphérique pour recevoir la tige de liaison de manière à autoriser une angulation relative adaptative entre la vis d'ancrage et l'élément de liaison intervertébrale.

Dans l'état de la technique de nombreux dispositifs d'ancrage du type à articulation sphérique ont été proposés. Ainsi, il est connu un dispositif d'ancrage osseux comportant un élément d'ancrage osseux tel qu'une vis munie d'une tête de réception d'un axe fileté sur lequel est destiné à être vissé un moyen de serrage d'une tige de liaison. Une articulation sphérique est aménagée entre l'élément d'ancrage osseux et l'axe fileté pour permettre une orientation pluridirectionnelle de l'axe fileté. Selon un exemple de réalisation, l'extrémité de l'axe fileté comporte un moyen de réception d'un ancillaire permettant de bloquer en rotation l'axe fileté lors de l'opération de vissage ou de dévissage de l'écrou de serrage sur l'axe fileté. Il apparaît ainsi que l'opération de serrage ou de desserrage de l'écrou sur l'axe fileté constitue une opération nécessitant l'utilisation de différents ancillaires, ce qui complique et rallonge l'opération de misé en place d'un système d'ostéosynthèse.

Il est connu, également, par le brevet US 5 628 740, un dispositif d'ancrage osseux comportant un élément d'ancrage osseux muni d'une tête de réception d'un axé fileté sur lequel est destiné à être vissé un moyen de serrage. Une articulation sphérique est aménagée entre l'élément d'ancrage osseux et l'axe fileté. L'axe fileté comporte deux tétons diamétralement opposés s'engageant dans des rainures aménagées dans la tête équipant l'élément d'ancrage osseux. Ces tétons coopèrent avec les rainures pour permettre le pivotement de l' axe fileté.

De même, le brevet US 5 725 528 décrit un dispositif d'ancrage osseux conforme au préambule de la revendication 1, notamment comportant un élément d'ancrage osseux muni d'une tête de réception d'un axe fileté sur lequel est destiné à être vissé un moyen de serrage, une articulation sphérique étant aménagée entre l'élément d'ancrage osseux et l'axe fileté pour permettre une orientation pluridirectionnelle de l'axe fileté.

Un tel dispositif ne permet pas de conserver une orientation pluridirectionnelle à l'axe fileté par rapport à l'élément d'ancrage osseux, tout en assurant une liaison efficace en rotation, entre l'élément d'ancrage osseux et l'axe fileté.

L'objet de l'invention vise donc à remédier aux inconvénients de l'art antérieur en proposant un dispositif d'ancrage osseux permettant d'assurer de manière simple et sûre, le vissage voire le dévissage d'un écrou de serrage sur l'axe fileté d'un tel dispositif d'ancrage osseux qui conserve, par ailleurs, sa fonction d'orientation pluridirectionnelle de l'axe fileté.

Pour atteindre de tels objectifs le dispositif d'ancrage osseux selon l'invention est conforme à la revendication 1.

Diverses autres caractéristiques ressortent de la description faite ci-dessous en référence aux dessins annexées qui montrent, à titre d'exemples non limitatifs, des formes de réalisation de l'objet de invention.

La **Fig. 1** est une vue schématique d'un exemple de réalisation d'un dispositif d'ancrage osseux selon l'invention.

La **Fig. 2** est une vue en coupe-élévation à plus grande échelle de l'exemple de réalisation illustré à la **Fig.1****.**

La **Fig. 3** est une vue partielle en perspective montrant un détail caractéristique de l'objet de l'invention.

Tel que cela apparaît sur les figures, l'objet de l'invention concerne un dispositif d'ancrage osseux **1 destiné** à être implanté dans le rachis. Un tel dispositif d'ancrage osseux **1** comporte un élément d'ancrage oiseux **2** au sens général, adapté pour être réalisé de différentes manières connues. L'élément d'ancrage osseux **2** peut se présenter sous la forme d'un crochet, d'une plaque; ou tel que représenté sur les dessins d'une vis d'ancrage. Cet élément d'ancrage osseux **2** est muni d'une tête de réception **3** pour un axe fileté **4** sur lequel est destiné à être vissé un moyen de serrage **5**. D'une manière classique, une articulation sphérique **6** est aménagée entre l'élément d'ancrage osseux **2** et l'axe fileté **4** pour permettre une orientation pluridirectionnelle de l'axe fileté **4** par rapport à l'élément d'ancrage osseux **2.**

Dans l'exemple illustré de réalisation, l'axe fileté **4** est pourvu à l'extrémité opposée de son extrémité libre, d'une rotule **7** de forme hémisphérique montée à l'intérieur d'un logement **8** de forme complémentaire pour constituer l'articulation sphérique **6.** Dans l'exemple illustré, le logement **8** est aménagé à l'intérieur de la tête de réception **3** qui se présente sous la forme d'un écrou de préhension pour un outil de vissage. Cette tête de réception **3** qui est par exemple rapportée en étant fixée sur l'élément d'ancrage osseux **2,** comporte une ouverture **9** s'ouvrant à l'intérieur du logement **8** pour autoriser le passage de l'axe fileté **4** et son débattement angulaire pluridirectionnel par rapport à l'élément d'ancrage osseux **2.** Bien entendu, il peut être prévu de réaliser la rotule **7** sur l'extrémité de l'élément d'ancrage osseux **2** et le logement **8** sur l'axe fileté **4.**

Conformément à l'invention, le dispositif d'ancrage osseux **1** comporte des moyens **10** de liaison en rotation entre l'élément d'ancrage osseux **2** et l'axe fileté **4.** En d'autres termes, l'élément d'ancrage osseux **2** et l'axe fileté **4** sont liés directement en rotation tout en conservant une orientation pluridirectionnelle à l'axe fileté **4** par rapport à l'élément d'ancrage osseux **2.** A cet égard, les moyens de liaison **10** sont réalisés en dehors de l'articulation sphérique **6.**

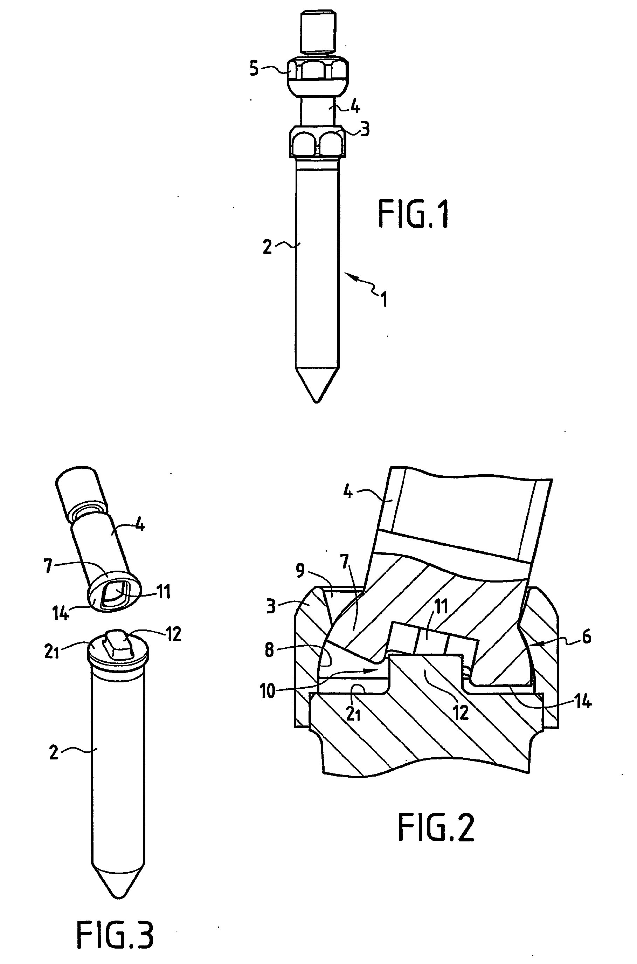

Tel que cela ressort plus précisément des **Fig. 2** et **3****,** les moyens de liaison en rotation **10** sont constitués par une forme géométrique ou empreinte femelle **11** coopérant avec une forme géométrique ou empreinte mâle **12** complémentaire. L'une des formes géométriques **11, 12** est aménagée sur la tête de réception **3** de l'élément d'ancrage osseux **2** tandis que l'autre forme géométrique est réalisée sur l'extrémité de l'axe fileté **4**.

Ces formes géométriques femelle **11** et mâle **12** selon l'invention sont dites complémentaires dans le sens où un effort de rotation exercé selon l'axe de l'élément d'ancrage osseux **2** ou de l'axe fileté **4** conduit à un blocage en rotation entre l'élément d'ancrage osseux **2** et l'axe fileté **4,** quelle que soit l'orientation relative entre ces deux pièces. Un tel blocage en rotation est obtenu directement entre l'élément d'ancrage osseux **2** et l'axe fileté **4** sans l'aide d'autres pièces. Il doit donc être considéré que les formes géométriques femelle **11** et mâle **12** peuvent présenter des sections droites transversales de formes identiques ou différentes. Dans l'exemple de réalisation illustré et tel que cela ressort de la **Fig. 3****,** les sections droites transversales des formes géométriques femelle **11** et mâle **12** sont sensiblement rectangulaires. Bien entendu, les formes géométriques femelle **11** et mâle **12** peuvent présenter des sections de différentes formes, telles qu'elliptiques, ovales, non circulaires avec au moins une arête, etc. La forme géométrique femelle **11** est réalisée par une cavité ou un logement tandis que la forme géométrique mâle **12** est réalisée par un téton ou une partie saillante.

Tel que cela ressort de ce qui précède, les formes géométriques femelle **11** et mâle **12** délimitent entre elles un jeu pour autoriser l'orientation pluridirectionnelle entre l'axe fileté **4** et l'élément d'ancrage osseux **2** tout en assurant une liaison en rotation entre les formes géométriques mâle et femelle.

De manière préférée, il est à noter que la face transversale **14** délimitant la rotule 7 et la face d'extrémité **2₁** de l'élément d'ancrage osseux **4** s'étendent en vis à vis et à distance l'une de l'autre pour autoriser l'orientation pluridirectionnelle de l'axe fileté **4.** D'une manière avantageuse, la face transversale **14** de la rotule **7** et/ou la face d'extrémité **2₁** de l'élément d'ancrage osseux **2** possède une forme convexe pour autoriser une telle orientation avec un encombrement limité.

L'une des formes géométriques, à savoir la forme géométrique mâle **12** dans l'exemple illustré est aménagée sur l'extrémité de l'élément d'ancrage osseux **2** tandis que la forme géométrique femelle **11** est aménagée à l'extrémité libre de l'axe fileté **4.** D'une manière plus précise, la forme géométrique femelle **11** est réalisée dans la face transversale **14** délimitant la rotule hémisphérique **7,** tandis que la forme géométrique mâle **12** est réalisée sur la face d'extrémité **2₁** de l'élément d'ancrage osseux **2.** Bien entendu, la position des formes géométriques mâle et femelle sur l'axe fileté et sur l'élément d'ancrage osseux peut être inversée.

Le dispositif d'ancrage **1** selon l'invention possède donc une articulation sphérique autorisant ainsi une angulation relative adaptative entre l'élément d'ancrage osseux **2** et un organe d'un système d'ostéosynthèse tel qu'une tige de liaison. La fixation de cette tige de liaison par l'intermédiaire d'un moyen de serrage **5** est réalisée de manière simple sans avoir recours à un ancillaire pour maintenir en position l'axe fileté **4** dans la mesure où ce dernier ne peut pas tourner en raison de l'ancrage de l'élément **2** et de la présence des moyens de liaison en rotation **10.**

## Revendications

1. Dispositif d'ancrage osseux comportant un élément d'ancrage osseux **(2)** muni d'une tête de réception **(3)** pour un axe fileté **(4)** sur lequel est destiné à être vissé un moyen de serrage **(5),** une articulation sphérique **(6)** étant aménagée entre l'élément d'ancrage osseux **(2)** et l'axe fileté et **(4)** pour permettre une orientation pluridirectionnelle de l'axe fileté, ledit dispositif d'ancrage osseux comportant des moyens **(10)** de liaison en rotation entre l'élément d'ancrage osseux **(2)** et l'axe fileté **(4)** constitués par une forme géométrique l'une des formes géométriques étant aménagée sur l'extremité de l'élément d'ancrage osseux **(2)** tandis que l'autre forme géométrique est réalisée sur l'extrémité de l'axe fileté **(4),** femelle **(11)** coopérant avec une forme géométrique mâle complémentaire **(12)**, lesdits moyens de liaison en rotation conduisant, lorsqu'un effort de rotation est exercé selon l'axe de l'élément d'ancrage osseux **(2)** ou de l'axe fileté **(4)**, à un blocage en rotation entre l'élément d'ancrage osseux **(2)** et l'axe fileté **(4)**, **caracterisé en ce que** les formes géométriques femelle **(11)** et mâle **(12)** présentent des sections droites transversales non circulaires et **en ce que** les formes géométriques femelle **(11)** et mâle **(12)** délimitent entre elles un jeu pour autoriser l'orientation pluridirectionnelle entre l'axe filité **(4)** et l'élément d'ancrage osseux **(2)** tout en assurant une liaison en rotation entre les formes géométriques mâle et femelle.

2. Dispositif d'ancrage osseux selon la revendication 1, **caractérisé en ce que** les moyens de liaison en rotation **(10)** sont aménagés en dehors de l'articulation sphérique **(6).**

3. Dispositif d'ancrage osseux selon la revendication 1 ou 2, **caractérisé en ce que** l'une des formes géométriques **(11, 12)** est aménagée sur une face d'extrémité **(2₁)** de l'élément d'ancrage osseux **(2),** en s'étendant l'intérieur d'un logement ouvert **(8)** aménagé dans la tête de réception **(3)** et recevant l'extrémité de l'axe fileté **(4)** réalisée, sous la forme d'une rotule **(7)** pour constituer l'articulation sphérique **(6),** ladite rotule étant pourvue sur sa face transversale **(14)** de l'autre forme géométrique.

4. Dispositif d'ancrage osseux selon la revendication 3, **caractérisé en ce que** la face transversale **(14)** de la rotule **(7)** et la face d'extrémité **(2₁)** de l'élément d'ancrage osseux **(2)** s'étendent à distance l'une de l'autre pour autoriser l'orientation pluridirectionnelle de l'axe fileté **(4).**

5. Dispositif d'ancrage osseux selon la revendication 4, **caractérisé en ce que** la face transversale **(14)** de la rotule et/ou de la face d'extrémité **(2₁)** de l'élément d'ancrage osseux **(2)** possède une forme convexe.

6. Dispositif d'ancrage osseux selon la revendication 3, **caractérisé en ce que** la 5 forme géométrique mâle **(12)** est réalisée sur l'extrémité de l'élément d'ancrage osseux **(2)** tandis que la forme géométrique femelle **(11)** est aménagée sur la rotule **(7).**

7. Dispositif d'ancrage osseux selon la revendication 1, **caractérisée en ce que** la tête de réception (3) forme un écrou de préhension pour un outil de vissage.

## Claims

1. A bone anchoring device comprising a bone anchoring element (2) provided with a head (3) for receiving a threaded shaft (4) on which a clamping means (5) is screwed, a spherical articulation (6) being provided between the bone anchoring element (2) and the threaded shaft (4) to allow multiple orientation of the threaded shaft, said bone anchoring device providing rotational linkage means (10), between the bone anchoring element (2) and the threaded shaft (4), comprised of a female geometrical form (11) cooperating with a complementary male geometrical form (12), one of the geometrical forms being provided on the end of the bone anchoring element (2) and the other geometrical form being provided on the end of the threaded shaft (4), said rotational linkage means leading, when a rotational force is exerted along the axis of the bone anchoring element (2) or the threaded shaft (4), to rotational locking between the bone anchoring element (2) and the threaded shaft (4), **characterised in that** the female (11) and male (12) geometrical forms present non circular transversal straight sections and **in that** the female (11) and male (12) geometrical forms delimit between them clearance in order to allow multiple orientation between the threaded shaft (4) and the bone anchoring element (2) while maintaining rotational linkage between the male and female geometrical forms.

2. The bone anchoring device according to claim 1, wherein the rotation linkage means (10) is provided outside the spherical articulation (6).

3. The bone anchoring device according to claim 1 or 2, **characterised in that** one of the geometrical forms (11, 12) is provided on an end face (2₁) of the bone anchoring element (2), extending within an open housing (8) provided in the receiving head (3) and receiving the end of the threaded shaft (4) made as a ball-and-socket joint (7) in order to form the spherical articulation (6), said ball-and-socket joint being provided with the other geometrical form on its transverse face (14).

4. The bone anchoring device according to claim 3, **characterised in that** the transverse face (14) of the ball-and-socket joint (7) and the end face (2₁) of the bone anchoring element (2) extend a distance from one another in order to allow multiple orientation of the threaded shaft (4).

5. The bone anchoring device according to claim 4, **characterised in that** at least one of the transverse face (14) of the ball-and-socket joint and the end face (2₁) of the bone anchoring element (2) has a convex shape.

6. The bone anchoring device according to claim 3, **characterised in that** the male geometrical form (12) is made on the end of the bone anchoring element (2) and **in that** the female geometrical form (11) is provided on the ball-and-socket joint (7).

7. The bone anchoring device according to claim 1, **characterised in that** the receiving head (3) forms a grip nut for a screwing tool.

## Patentansprüche

1. Knochenverankerungsvorrichtung mit einem Knochenverankerungselement (2), das mit einem Aufnahmekopf (3) für eine Gewindeachse (4) versehen ist, auf die ein Spannmittel (5) aufgeschraubt werden soll, wobei ein Kugelgelenk (6) zwischen dem Knochenverankerungselement (2) und der Gewindeachse (4) angeordnet ist, um eine mehrdirektionale Ausrichtung der Gewindeachse zu ermöglichen, wobei die Knochenverankerungsvorrichtung Mittel (10) zur Drehverbindung zwischen dem Knochenverankerungselement (2) und der Gewindeachse (4) umfaßt, die von einer geometrischen Aufnahmeform (11), welche mit einer ergänzenden geometrischen Einsteckform (12) zusammenwirkt, gebildet sind, wobei die eine der geometrischen Formen am Ende (3) des Knochenverankerungselements (2) angeordnet ist, während die andere geometrische Form am Ende der Gewindeachse (4) angeordnet ist, wobei die Drehverbindungsmittel dann, wenn entlang der Achse des Knochenverankerungselements (2) oder der Gewindeachse (4) eine Drehkraft ausgeübt wird, zu einer Drehblockierung zwischen dem Knochenverankerungselement (2) und der Gewindeachse (4) führen, **dadurch gekennzeichnet, daß** die geometrische Aufnahmeform (11) und die geometrische Einsteckform (12) nicht kreisförmige Querschnitte aufweisen und daß die geometrische Aufnahmeform (11) und die geometrische Einsteckform (12) zwischen sich ein Spiel begrenzen, um die mehrdirektionale Ausrichtung zwischen der Gewindeachse (4) und dem Knochenverankerungselement (2) zuzulassen, und gleichzeitig eine Drehverbindung zwischen den geometrischen Aufnahme- und Einsteckformen gewährleisten.

2. Knochenverankerungsvorrichtung nach Anspruch 1, **dadurch gekennzeichnet, daß** die Drehverbindungsmittel (10) außerhalb des Kugelgelenks (6) angeordnet sind.

3. Knochenverankerungsvorrichtung nach Anspruch 1 oder 2, **dadurch gekennzeichnet, daß** die eine der geometrischen Formen (11, 12) an einer Endseite (2₁) des Knochenverankerungselements (2) angeordnet ist und sich dabei innerhalb einer offenen Aufnahme (8) erstreckt, die in dem Aufnahmekopf (3) ausgebildet ist und das Ende der Gewindeachse (4) aufnimmt, welches in Form eines Kugelkopfes (7) ausgebildet ist, um das Kugelgelenk (6) zu bilden, wobei der Kugelkopf an seiner Querseite (14) mit der anderen geometrischen Form versehen ist.

4. Knochenverankerungsvorrichtung nach Anspruch 3, **dadurch gekennzeichnet, daß** die Querseite (14) des Kugelkopfes (7) und die Endseite (2₁) des Knochenverankerungselements (2) im Abstand voneinander verlaufen, um die mehrdirektionale Ausrichtung der Gewindeachse (4) zu ermöglichen.

5. Knochenverankerungsvorrichtung nach Anspruch 4, **dadurch gekennzeichnet, daß** die Querseite (14) des Kugelkopfes und/oder die Endseite (2₁) des Knochenverankerungselements (2) eine konvexe Form aufweist.

6. Knochenverankerungsvorrichtung nach Anspruch 3, **dadurch gekennzeichnet, daß** die geometrische Einsteckform (12) an dem Ende des Knochenverankerungselements (2) ausgebildet ist, während die geometrische Aufnahmeform (11) an dem Kugelkopf (7) ausgebildet ist.

7. Knochenverankerungsvorrichtung nach Anspruch 1, **dadurch gekennzeichnet, daß** der Aufnahmekopf (3) eine Greifmutter für ein Schraubwerkzeug bildet.
